Europäisches Patentamt

European Patent Office

Office européen des brevets

(11) Publication number: **0 111 087**
**B1**

(12) **EUROPEAN PATENT SPECIFICATION**

(45) Date of publication of patent specification: **16.06.87**

(21) Application number: **83109990.8**

(22) Date of filing: **06.10.83**

(51) Int. Cl.⁴: **A 61 M 1/00**

(54) **Medical suction drainage apparatus.**

(30) Priority: **15.10.82 US 434681**

(43) Date of publication of application:
**20.06.84 Bulletin 84/25**

(45) Publication of the grant of the patent:
**16.06.87 Bulletin 87/25**

(84) Designated Contracting States:
**AT BE CH DE FR GB IT LI LU NL SE**

(56) References cited:
**EP-A-0 096 195**
**GB-A-1 540 865**
**GB-A-2 082 071**
**US-A-3 750 692**
**US-A-4 112 948**
**US-A-4 195 633**

(73) Proprietor: **SORENSON RESEARCH CO. INC.**
**4455 Atherton**
**Salt Lake City Utah 84107 (US)**

(72) Inventor: **Johnson, Robert H.**
**490 East 10600 South**
**Sandy, UT 84070 (US)**

(74) Representative: **Modiano, Guido et al**
**MODIANO, JOSIF, PISANTY & STAUB Modiano**
**& Associati Via Meravigli, 16**
**I-20123 Milan (IT)**

Courier Press, Leamington Spa, England.

## Description

### 1. Field of the invention

The present invention relates to drainage of fluids from body cavities and, more particularly, to an apparatus for use in draining fluids from the chest cavity.

### 2. The prior art

The thoracic or chest cavity is a closed structure essentially formed by the thoracic skeleton and muscles. The interior of the thoracic cavity is partitioned by the mediastinum, which consists of connective tissue which surrounds and holds together the esophagus, trachea, heart, aorta and other major vessels. The mediastinum divides the interior of the thoracic cavity into lung chambers (called pleural cavities), each of which contains one of the lungs.

The lungs are composed of elastic fibers which expand and contract during the normal breathing process. Expansion of the lung occurs during inhalation. When a person inhales, the diaphragm, which is mostly muscle, contracts and pulls downward. At the same time the chest muscles pull the chest wall up and out, with the result that the two chambers inside the thoracic cavity are expanded. The expansion of the two chambers in the thoracic cavity creates a negative pressure which exerts a pull on the lungs causing the lungs to expand and thereby allowing air to be drawn into the lungs. Similarly, during exhalation the rib cage and diaphragm contract, reducing the negative pressure in the two chambers which reduces the force on the lungs allowing them to contract so that air is exhaled.

When the chest wall is penetrated, either by surgical intervention or accidentally, air is permitted to enter the pleural cavity. As air enters negative pressure is no longer exerted on the lung, which results in pneumothorax, a condition in which the elastic fibers of the lung recoil or collapse. If air continues to leak into the pleural cavity a condition known as tension pneumothorax may develop. In this condition the pressure within the pleural cavity rises to the point where it causes the mediastinum, including the heart and the other major vessels supported in the mediastinum, to be pushed towards the unaffected lung. Should the pressure become great enough it can collapse the unaffected lung and interfere with heart action and can thus lead to death within a few minutes.

In order to restore normal breathing following pneumothorax, the air and other fluids which have entered the pleural cavity must be removed from around the lung. This is typically accomplished by inserting one or more chest catheters into the pleural cavity and then connecting the catheters to a drainage system which is used to collect the fluids drained from the pleural cavity.

There are several types of prior art drainage systems which have been used for this purpose. For example, one type of system utilizes gravity to effect drainage of the fluids from the pleural cavity. In this type of system, a bottle is placed below the level of the patient's chest. The bottle is closed at its top by a rubber stopper through which a drainage tube is inserted. The drainage tube is attached at one end to the catheter inserted into the patient's chest. The other end of the drainage tube extends through the stopper to a point near the bottom of the bottle. A sterile liquid such as saline solution is used to fill the bottle to a point which covers the end of the drainage tube. The sterile liquid is intended to act as a seal or one-way check valve which prevents air from moving back up through the drainage tube to the patient's chest. The bottle is also vented to atmosphere through the rubber stopper so that when the bottle is placed below the level of the patient's chest, gravity will effect drainage of fluid from the pleural cavity into the drainage bottle.

This system will not work if the lung is fully collapsed, and it has the further disadvantage that it is difficult to overcome air leaks around the catheter. Moreover, at times there may be a major fluid leak which requires additional drainage capacity. Thus, suction drainage systems have also been used in the prior art to obtain increased drainage capacity. Suction drainage systems typically include a water manometer which is connected to a source of suction and which controls the level of suction applied to the pleural cavity of the patient, since an uncontrolled level of suction may damage the surrounding tissue. The manometer bottle is in turn connected to a bottle which contains a water seal similar to the type of water seal used in a gravity drainage system. The bottle containing the water seal may then be also connected to a third bottle which is used as the drainage bottle for collecting the fluids that are drained from the patient's chest cavity.

One of the disadvantages experienced with the use of this type of three bottle suction drainage system is the rather complicated procedure for setting up the system and interconnecting the three bottles. Also, the system is somewhat inconvenient to use because the bottles must be rinsed, washed and sterilized before they can be used again on other patients.

A more recent system which overcomes some of the diadvantages of the three bottle suction drainage system is illustrated and described in U.S. Patent Nos. 3,363,626 and 3,363,627. These patents describe a unitary or consolidated "three bottle" apparatus which is constructed of plastic and which is disposable. Rather than using separate bottles, the apparatus replaces the bottles with separate chambers that are formed as part of a single container. The apparatus retains the basic concept of the three bottle suction drainage system because one of the chambers of the apparatus is used as a manometer, and is connected to a second chamber which is used as a water seal. The second chamber is connected to a third chamber which is used as the drainage chamber for receiving fluids drained from the patient's chest cavity.

While the apparatus described in these patents simplifies the set up procedure of the basic three bottle suction drainage system and provides for convenient disposal of the system after each use, this apparatus is relatively complicated in its structure and is expensive to manufacture and use. Moreover, like the gravity drainage system and the three bottle suction drainage system, the apparatus of these patents continues to rely upon the use of an underwater seal which is intended to prevent fluids from re-entering the patient's pleural cavity. However, in practice, an underwater seal does not always prevent fluids from re-entering the patient's chest cavity. For example, if the patient's bronchial tubes are blocked the patient must take deeper breaths in order to expand the lungs to permit air flow around this blockage. When the patient gasps for air or continually takes these kinds of deep breaths, a sufficiently high negative pressure may be developed in the pleural cavity that the liquid used to provide the water seal may be sucked back through the tube and catheter and into the pleural cavity. This obviously increases the risk of contamination to the patient, as well as hampering recovery of the patient's normal respiration.

Known from GB—A—2082071 and GB—A—1540865 are a chest drainage apparatus and a surgical drainage device respectively, each comprising the combination cited in the pre-characterizing clause of claim 1.

Also known from US—A—4122948 is a surgical drainage system including a collection chamber and a manometer in fluid location with a seal element located upstream of the collection chamber.

Although this configuration will prevent backflow of fluids from the collection chamber into the patient's chest cavity, significant disadvantages are inherent in this positioning of the seal. The most important disadvantage is that all fluids drained from the chest must pass through the seal. Since the seal employs separable abutting surfaces, retention of both flexibility and separability is essential to its function.

Although these properties can easily be maintained if the fluids aspirated from the chest cavity are water and air, it will frequently be the case that blood, serum and similar materials will also be aspirated, and these materials pose a substantial risk that the seal may become clogged, stuck, or encrusted with debris, defeating the essential purpose of the seal and causing malfunction of the unit. In addition, this configuration presents the opportunity for such materials to enter the manometer chamber, eliminating the reliability of the indicated vacuum level. Finally, because the manometer is in fluid communication with the seal on the upstream side of the collection chamber, and thus in field communication with the patient's chest cavity, the manometer cannot be open to the ambient air. Thus, the manometer is equilibrated to atmospheric pressure only once, at the time the unit is connected. Thereafter, the unit will be unable to compensate the changes in ambient pressure—which can easily fluctuate by several inches of water in a day—unless it is disconnected from the patient.

Thus, what is needed in the art is a suction drainage system which is less expensive and can be economically disposed of after each use and which also overcomes the disadvantages of the prior art type of systems with their attendant disadvantages. Such an invention is described and obtained herein.

Summary and objects of the invention

One important object of the present invention is to provide an apparatus for draining fluids from the body cavity of a patient which includes structure for effectively sealing the drainage system to prevent backflow of drained fluids but which seal is accomplished without the use of an underwater seal.

Another important object of the present invention is to provide an apparatus for draining fluids from a body cavity which is simple in its construction, compact, easy to handle and which can be economically disposed of after each use.

Yet another important object of the present invention is to provide an apparatus which combines in a simple and effective way the structure for accomplishing pressure regulation and sealing of a suction drainage system.

A not least object of the invention is to provide an apparatus for draining fluids from a body cavity, which is completely reliable in use, even in the event of blood, serum or similar matter being aspirated.

These and other objects which will become apparent hereinafter are achieved by the invention, according to one aspect whereof, there is provided a suction drainage system for draining liquid and gaseous fluids from a body cavity, said system comprising:

means (22) for collecting said fluids drained from said body cavity;

means (12, 14, 16, 18) for establishing a first flow path (23) between said body cavity and said means (22) for collecting said drained fluids;

means (26, 28, 28a, 28b, 32, 36, 40, 41) for establishing a second flow path (37) between said means (22) for collecting said fluids and a source of vacuum;

means (46) comprising inlet (46a) and outlet (46b) ends and being disposed in said second flow path (37) for providing one-way flow of fluid from said means (22) for collecting said fluids to a suction control chamber (30); and

a suction control chamber (30) having inlet (32) and outlet (36) openings connected in series in said second flow path (37), said means (46) for providing one-way flow being disposed within said suction control chamber (30) in series with said inlet opening (32), said suction control chamber (30) including a liquid (59) disposed therein, (characterized in that) said liquid is a sterile liquid and in that the outlet end (46b) of said means (46) for providing one-way flow is disposed under the level of said sterile liquid (59) in contact therewith.

According to another aspect of the invention there is provided a suction control chamber (30) for use in applying suction to a body cavity through a suction collection container (22), said suction control chamber (30) comprising:

an inlet (32) and an outlet (36), said inlet (32) being adapted to be connected in fluid flow communication with said collection container (22), said outlet being adapted to be connected in fluid flow communication with a source of suction;

check valve (46) having inlet (46a) and outlet (46b) ends and being disposed at the inlet (32) of said suction control chamber (30) to permit one-way flow of fluids from said inlet (32) into said suction control chamber (30), and

pressure monitoring means (64, 66, 68) disposed in said suction control chamber (30) for providing an indication of the pressure therein, said suction control chamber (30) including a liquid (59) disposed therein, (characterized in that) said liquid is a sterile liquid and in that the outlet end (46b) of said check valve (46) means is disposed under the level of said sterile liquid (59) in contact therewith.

Brief description of the drawings

The foregoing and other features, advantages and objects of the present invention will become more fully apparent from the following detailed description of specific embodiments thereof, especially when taken in conjunction with the accompanying drawings, wherein:

Figure 1 is a schematic illustration of the apparatus of the present invention connected to a patient to effect drainage of the chest cavity;

Figure 2 is a perspective illustration of the suction drainage system of the present invention which illustrates the suction control chamber connected to a typical suction collection apparatus;

Figure 2a is an enlarged perspective illustration in partial cross-section, illustrating a one-way valve that may be used to prevent loss of vacuum in the suction collection container;

Figure 3 is a view in vertical section of the suction control chamber of the present invention, and also showing portions of the suction collection container in partial cross-section;

Figure 4 is a view in vertical section of a flow restrictor which can be substituted for the suction control valve shown in the apparatus in Figure 3; and

Figure 5 is a view in vertical section which illustrates an enlarged view of the structure of the apparatus of the present invention which may be used in measuring the level of suction which is applied.

Detailed description of the preferred embodiments

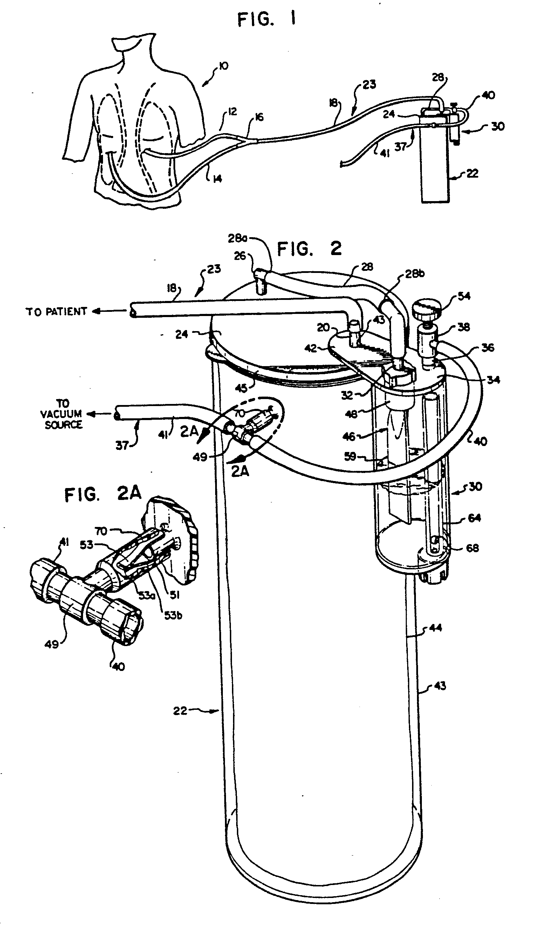

Referring specifically to Figure 1 of the accompanying drawings, a patient 10 is schematically illustrated with two drainage tubes 12, 14 connected in flow communication with his thoracic cavity. The number of tubes so used depends upon the nature of the procedure and is not a limiting feature of the present invention. The drainage tubes 12, 14 are connected to respective legs of a Y-connector 16 so as to feed a common flow tube 18 connected to the stem of the connector 16. Flow tube 18 is connected to a fitting 20 (see Figure 2) which projects upwardly from the cover 24 of a suction collection container generally designated at 22, to form a first flow path 23. Also projecting through cover 24 is a second fitting 26 (see Figure 2) to which a further flow tube 28 has one of its ends 28a connected. The other end 28b of tube 28 is connected to an inlet fitting 32 projecting upwardly from the top wall or cover 34 of a suction control chamber designated at 30. An outlet fitting 36 also projects upwardly from cover 34 and is connected to an adjustable control valve 38. Inlet fitting 32 and outlet fitting 36 are connected in series to form part of a second flow path 37 through which negative or suction pressure is adjustably applied to the interior of suction control chamber 30 by means of valve 38, which is connected by tubes 40 and 41 to a negative pressure or vacuum source (not shown).

The relatively small suction control chamber 30 can be mounted on the larger suction collection container 22 by means of the cover 34. The cover 34 (see Figure 2) includes a horizontal projection 42 which engages fitting 20 through aperture 43. As hereinafter more fully described, suction applied to chamber 30 is transmitted to container 22 via tube 28. From container 22 the suction is applied to the chest cavity of patient 10 via tubes 18, 12 and 14. Liquid drawn through tubes 12, 14 and 18 is thus suctioned into and collected in container 22 while drained gases pass above the collected liquid, through tube 28 to chamber 30 where they are exhausted through tubes 40 and 41 by the vacuum source.

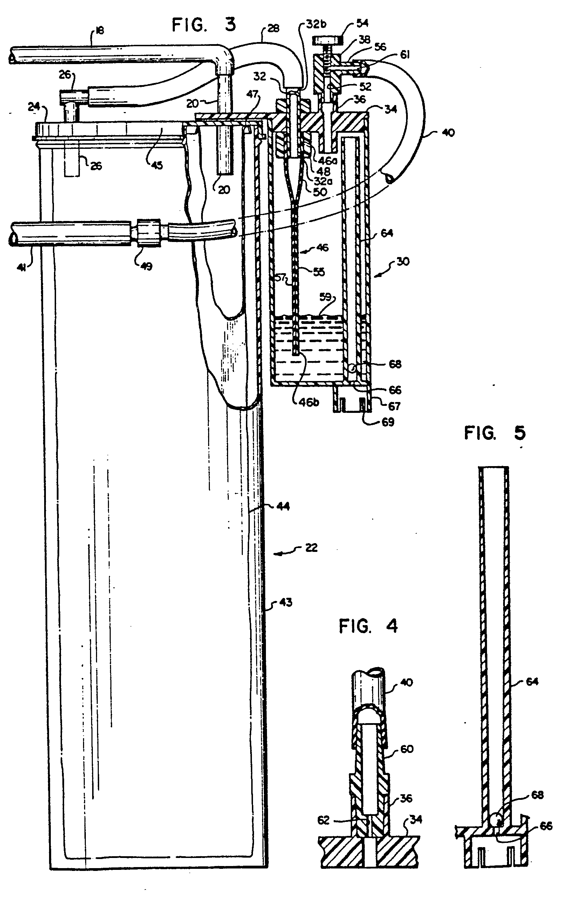

Referring more specifically to Figures 2 and 3, the suction collection container generally designated at 22 may be any suitable type of suction collection apparatus for effecting thoracic drainage, such as for example the apparatus illustrated and described in U.S. Patent No. 3,719,197 which is incorporated herein by reference. Briefly described, the suction collection container 22 includes a rigid outer canister 43 that is cylindrical in shape, although the shape is not critical. The rigid outer canister 43 is enclosed at its top by a cover 24 which has a depending flange 45 for airtight engagement over the upper open end of the canister 43. A plastic receiver or canister liner 44 depends from the cover 24 and is fused or otherwise secured to the underside of the cover 24 entirely around the upper periphery of the liner 44 as indicated at 47 (see Figure 3). The cover 24 is preferably of a relatively rigid plastic material, while the liner or receiver 44 which depends from the cover 24 is preferably a flexible thermoplastic material. The securement of the upper end portion of the liner 44 to the cover 24 is completely air-tight and positive. The liner receiver 44 is therefore completely sealed except for fittings 20 and 26, which may conveniently be molded as part of the cover 24 and which project from the cover 24 into the interior of the liner receiver 44.

The outer canister 43 has a T fitting 49 (see also figure 2a) secured to nipple 51 in the wall of the canister 43. As shown best in Figures 2 and 2a, one arm of the T fitting 49 is connected by tube 40 to the outlet fitting 36 of the control valve 38 on the suction control chamber 30. The other arm of the T fitting 49 is connected to the tubing 41 connected to the source of suction (not shown) used to effect drainage of the patient's chest cavity. A resilient, one-way flutter valve 53 is attached at the nipple 51 and is enclosed by the collar portion 70 of T fitting 49. If suction through tube 41 is terminated, a positive pressure is established across valve 53 from its outlet end 53a to its inlet end 53b, which serves to help seal valve 53. Valve 53 thus prevents the loss of vacuum from the inside of canister 43 and subsequent collapse of the liner 44 if the source of vacuum is disconnected for any reason.

The vacuum or negative pressure applied through tube 41 is communicated through the collar portion 70 of T fitting 49 and through valve 53 to the interior of the rigid canister 43. As described in the aforementioned patent, in this manner negative pressure or vacuum created inside the interior of the receiver or liner 44 is countervailed by the vacuum that is established inside of the canister 43 but outside the liner 44, thus preventing collapsing of the liner receiver 44 during the time that the vacuum is applied.

Advantageously, since the liner receiver 44 is heat sealed or otherwise secured to the cover 24 once the liner has been completely filled with the fluids drained from the patient's chest cavity, the cover 24 and liner receiver 44 may simply be removed and disposed of intact. This eliminates the need for having to wash and resterilize the suction collection container 22 and also eliminates additional risk of contamination to nurses and other hospital staff.

The specific structure and operation of the presently preferred embodiment of the suction control chamber 30 of the present invention is best illustrated and described with reference to Figure 3. Inlet fitting 32 extends through the cover 34 of chamber 30 and has its interior end engaged within one end of an elastomeric duck-bill member generally designated at 46. Duck-bill member 46 is a radially flattened, elongated elastomeric tube which, in its unflexed state, prevents axial flow therethrough because its opposed flattened surfaces 55, 57 abut one another. The ingress end 46a of member 46 is stretched over the interior end 32a of fitting 32 and may be annularly clamped thereto by a clamping ring 48, or bonded directly to fitting 32. The inlet opening 32b of fitting 32 thus communicates with the interior of the resiliently expanded portion 50 of the member 46. Member 46 can be made of any elastomeric material and, in the preferred embodiment, is made of natural rubber. Clamping ring 48 is made of latex or other similar material suitable for clamping the ingress end 46a of member 46 about fitting 32.

Outlet fitting 36 is secured to the external side of cover 34 to valve 38 by means of a friction fit or the like. Valve 38 may be any suitable metering valve and, in the preferred embodiment, includes a threaded interior bore 52. A screw 54 threadingly engages bore 52. An outlet fitting 56 is adapted to be connected to tube 40 and has a throughbore 61 that communicates with the interior bore 52. By adjusting the depth of screw 54 in bore 52, the flow restriction between fittings 56 and 36 can be varied so as to adjust the suction or negative pressure applied to the interior of suction control chamber 30.

Alternatively, as illustrated in Figure 4, the valve 38 may be replaced by a flow restrictor 60 which is adapted to engage fitting 36 in a friction fit, or which may be bonded thereto. Flow restirctor 60 has a flow path which includes a narrowed portion 62 which restricts gaseous flow and thereby limits the suction pressure applied to chamber 30.

In practice, it has been found that the size of the restriction provided by bore 62 (see Figure 4) or screw 54 (see Figure 3) should be designed so that gaseous flow through the restriction will be maintained at sonic velocity over a range of absolute line pressures in tube 40 from approximately zero to one-half an atmosphere. For example, in one presently preferred embodiment, the restrictor 62 is sized at 0,1181 cm (.0465 in.), which causes a constant flow rate which in combination with aperture 66 results in an essentially constant negative pressure of about 20 cm of water using typical hospital vacuum wall outlets or portable vacuum pumps. This level of suction can be safely applied to the pleural cavity without damaging surrounding tissue.

With continued reference to Figure 3, the pressure within suction control chamber 30 is monitored by means of a pressure monitoring tube 64. Tube 64 preferably has gradations (not shown) spaced longitudinally along its exterior and calibrated in units of pressure. The tube 64 is oriented vertically within suction control chamber 30 and has its lower end communicating with the ambient pressure outside of chamber 30 by means of a small aperture 66 located in the bottom wall of the chamber 30. Aperture 66 is sized so that it is slightly larger than the flow restriction provided by screw 54 or restrictor tube 60. Using the example noted above wherein restrictor 62 is 0,1181 cm (.0465 in.), a suitable size for aperture 66 would be 0,1981 cm (.078 in.).

In the preferred embodiment the tube 64 is molded integrally with the bottom wall of chamber 30, with the lower end of the tube disposed concentrically about aperture 66. A round projection 67 having one or more slots 69 is formed on the bottom of chamber 30 and surrounds the aperture 66. The projection 67 prevents occlusion of the aperture 66 by a finger or by other objects.

A ball 68 or other relatively light-weight pressure responsive indicator is disposed within tube 64, which has its upper end open to the interior of chamber 30. Ball 68 is typically made of polypropylene or similar material. When the pressure in chamber 30 is reduced by the applied suction to a level below ambient pressure, air rushes in through aperture 66 causing ball 68 to rise in tube 64. The level attained by the ball corresponds to the magnitude of the pressure differential across tube 64 and hence, reflects the pressure level within chamber 30. The calibrated gradations (not shown) on tube 64 provide a reading of that pressure as a function of the height attained by the ball within tapered tube 64.

When negative pressure is introduced into the suction control chamber 30 through valve 38, a positive pressure differential is developed across duck-bill member 46 from its upper ingress end 46a to its lower egress end 46b. Gas drawn through fitting 32, which is suctioned from the patient's chest cavity via tube 28, collection container 22 and and tube 18, forces a radial expansion of member 46 to permit flow therethrough. This gas is then evacuated from chamber 30 via fitting 36. If the pressure in chamber 30 should exceed the pressure in liner 44, a negative pressure differential is created between upper ingress end 46a and lower egress end 46b. Duck-bill member 46 is thereby radially compressed, rendering the seal against backflow to liner 44 even stronger than that provided by the natural resilience of member 46. It will therefore be appreciated that member 46 serves as a mechanical check valve which eliminates the need for a water seal.

However, for many applications it may be desirable to fill chamber 30 with a sterile saline solution or other sterile liquid 59 up to a level which is above the egress end 46b of duck bill member 46 (a check valve), as illustrated in Figures 2 and 3. The liquid 59 is not provided to effect a seal but it permits drained gases to be visually observed as they bubble through the liquid 59. In the absence of such a liquid, the gases will be efficiently drained from the pleural cavity without danger of backflow; however, there would be no visible indication of such gaseous fluid drainage. In addition, the liquid 59 provides a means of permitting detection of leaks in the system between the patient and the check valve 46. Such a leak would be easily detected by the excessive bubbling from the egress end of member 46 through the liquid 59. Importantly, however, the liquid 59 cannot be suctioned back into the liner 44 or patient's cavity because of the presence of the mechanical check valve provided by duck bill member 46.

In order to provide an example of the size of the suction control chamber and its components, a set of dimensional values is provided hereinbelow. It must be stressed, however, that the dimensions are by way of example only and are not to be construed as limiting the scope of the invention. In this example, chamber 30 is a transparent plastic cylinder having an inside diameter of 4,293 cm (1.690 inches), an outside diameter of 4,445 cm (1.750 inches), and an axial length of 11,24 cm (4.425 inches). The base of the chamber is preferably formed integrally with the cylinder and has an axial depth of 0,381 cm (0.150 inches). Pressure monitoring tube 64 (see Figure 5) is preferably formed integrally with the chamber base and has an axial length of 10,16 cm (4.000 inches). The outer diameter of tube 64 is 1,041 cm (0.410 inch) and the inner diameter tapers from 0,635 cm (0.250 inch) at its lower end to 0,889 cm (0.350 inch) at its upper end. Duck bill member 46 is 10,16 cm (4.000 inches) long, 2,032 cm (0.800 inch) wide and 0,157 cm (0.062 inch) thick. A typical range of pressure calibration of tube 64 is between zero to twenty centimeters of water.

The invention may be embodied in other specific forms without departing from its essential characteristics. The described embodiment is to be considered in all respects only as illustrative and not restrictive and the scope of the invention is, therefore, indicated by the appended claims rather than by the foregoing description. All changes which come within the meaning and range of equivalency of the claims are to be embraced within their scope.

**Claims**

1. A suction drainage system for draining liquid and gaseous fluids from a body cavity, said system comprising:

means for collecting said fluids drained from said body cavity;

means for establishing a first flow path between said body cavity and said means for collecting said drained fluids;

means for establishing a second flow path between said means for collecting said fluids and a source of vacuum;

means comprising inlet and outlet ends and being disposed in said second flow path, for providing one-way flow of fluid from said means for collecting said fluids to a suction control chamber; and

a suction control chamber having inlet and outlet openings connected in series in said second flow path, said means for providing one-way flow being disposed within said suction control chamber in series with said inlet opening, said suction control chamber including a liquid disposed therein (characterized in that) said liquid is a sterile liquid and in that the outlet end of said means for providing one-way flow is disposed under the level of said sterile liquid in contact therewith.

2. A suction control chamber for use in applying suction to a body cavity through a suction collection container, said suction control chamber comprising:

an inlet and an outlet, said inlet being adapted to be connected in fluid flow communication with said collection container, said outlet being adapted to be connected in fluid flow communication with a source of suction;

check valve means, comprising inlet and outlet ends and being disposed at the inlet of said suction control chamber for providing one-way flow of fluids from said inlet into said suction control chamber, and

pressure monitoring means disposed in said suction control chamber for providing an indication of the pressure therein, said suction control chamber including a liquid disposed therein, (characterized in that) said liquid is a sterile liquid and in that the outlet end of said check valve means is disposed under the level of said sterile liquid in contact therewith.

3. The suction drainage system according to claim 1 wherein said means (46) for providing one-way flow comprise an elastomeric member having an inlet (46a) and an outlet (46b), and having two normally abutting opposed surfaces (55, 57) which are separable in response to application of a positive differential pressure established across said elastomeric member from its inlet (46a) to its outlet (46b).

4. The suction drainage system according to claim 1 wherein the gaseous fluids flowing from said means (46) for providing one-way flow are discharged through said sterile liquid (59) to the ambient pressure.

5. An apparatus according to claim 1 or 4 wherein said suction control chamber (30) includes an adjustable valve means (38, 52, 54), connected to said outlet opening (36) of said suction control chamber (30) for providing an adjustable restrictor (52, 54) at said outlet opening (36), and means (66) for venting said suction control chamber (30) to the ambient pressure.

6. The suction drainage system according to claim 5 wherein said valve means (38) includes a threaded bore (52) and a screw (54) which engages said threaded bore (52).

7. The suction drainage system according to claim 1 wherein said suction control chamber (30) includes a flow restrictor (60, 62) connected to said outlet opening (36) and means (66) for venting said suction control chamber (30) to the ambient pressure.

8. The suction drainage system according to claims 5 or 7 wherein said restrictor (52, 54, 60, 62) is sized so that gaseous flow through the restrictor (52, 54, 60, 62) is maintained at sonic velocity over a range of absolute pressure from zero to one-half an atmosphere.

9. The suction drainage system according to claim 7 wherein said means (66) for venting the suction control chamber (30) is sized slightly larger than said flow restrictor, whereby said flow restrictor (60, 62) and said means (66) for venting the suction control chamber (30) will provide in combination an essentially constant level of negative pressure inside said suction control chamber (30).

10. The suction drainage system according to claims 1, 4, 5, 7 or 9 further comprising pressure monitoring means (64, 68) disposed in said suction control chamber (30) for providing an indication of the pressure in said suction control chamber (30).

11. The suction drainage system according to claim 9 wherein said pressure monitoring means comprises:

a hollow tapered tube (64) having first and second ends, said first end having an aperture (66) which communicates with ambient pressure external to said suction control chamber (30), said second end being open and disposed within said suction control chamber (30) and

a movable, pressure-responsive indicator (68) disposed in said tube (64) for assuming a position between said first and second tube (68) ends which is proportional to the differential between said ambient pressure and the pressure within said suction control chamber (30).

12. The suction drainage system according to claim 11 wherein said pressure responsive indicator comprises a ball (68) constructed and arranged to move in said tube (64) in response to said pressure differential.

13. The suction drainage system according to claim 1 wherein said means (22) for collecting said drained fluids which comprises:

a rigid outer canister (43);

a disposable liner receiver (44) inside of said canister (43) said liner receiver having a cover (24); and

means (40, 41, 49, 51, 70) for communicating negative pressure from said vacuum source to both the inside of said liner receiver (44) and to the inside of said canister (43) so as to maintain the liner receiver (44) expanded when vacuum is applied to the system.

14. The suction drainage system according to claim 13 further comprising a one-way valve (53, 53a, 53b) disposed downstream from said means (40, 49, 51, 70) for communicating said negative pressure to the inside of said canister (43) thereby preventing the loss of vacuum within said canister (43) upon termination of suction from said vacuum source.

15. The suction drainage system according to claim 1, wherein said suction control chamber (30) comprises means (34) for removably attaching said suction control chamber (30) to said means (22) for collecting said drained fluids.

16. The suction drainage system according to claim 11, further comprising means (67, 69) for precluding said aperture (66) from being occluded by a finger or other like object.

17. The suction control chamber according to claim 2 wherein said pressure monitoring means (64, 66, 68) comprises:

a hollow tapered tube (64) having first and second ends, said first end having an aperture (66) which communicates with ambient pressure external to said suction control chamber (30), said second end being open and disposed within said suction control chamber (30); and

a movable, pressure-responsive indicator (68) disposed in said tube (64) for assuming a position between said first and second tube ends which is proportional to the differential between said ambient pressure and the pressure within said suction control chamber (30).

18. The suction control chamber according to claim 2 further comprising means (34) for removably mounting said suction control chamber (30) on said collection container (22).

19. The suction control chamber according to claim 2 further comprising adjustable valve means (38, 52, 54), connected to said outlet (36) of said suction control chamber (30), for providing an adjustable restrictor at said outlet (36) opening, and means (66) for venting said suction control chamber (30) to the ambient pressure.

20. The suction control chamber according to claim 2 further comprising a flow restrictor (60) connected to said outlet (36) of said suction control chamber (30) and means (66) for venting said suction control chamber (30) to the ambient pressure.

21. The suction control chamber according to claim 20 wherein said flow restrictor (60) is sized so that gaseous flow through said restrictor (60) is maintained at sonic velocity over a range of absolute pressure from zero to one half an atmosphere.

22. The suction control chamber according to claim 2 wherein said check valve means (46) comprises an elastomeric member having two normally abutting opposed surfaces (55, 57) which are separable in response to a positive pressure differential established across the check valve means (46).

23. The suction control chamber according to claim 19 wherein said adjustable valve means include a threaded bore (52) and a screw (54) which engages said threaded bore (52).

24. The suction control chamber according to claim 21 wherein said means (66) for venting said suction control chamber (30) is sized slightly greater than said restrictor (60), whereby said restrictor (60) and said means (66) for venting the suction control chamber (30) will provide in combination an essentially constant level of negative pressure inside said suction control chamber (30).

**Patentansprüche**

1. Absaugdränagesystem zum Dränen von flüssigen und gasformigen Fluiden aus einer Körperhöhle, umfassend

eine Einrichtung zum Sammeln der aus der Körperhöhle gedränten Fluide,

Mittel zum Bilden eines ersten Strömungsweges zwischen der Körperhöhle und der Sammeleinrichtung für die gedränten Fluide,

Mittel zum Bilden eines zweiten Strömungsweges zwischen der Sammeleinrichtung für die Fluide und einer Vakuumquelle,

eine im zweiten Strömungsweg angeordnete Einrichtung mit Ein- und Auslaßenden zum Ausbilden einer Einwegströmung von Fluid aus der Sammeleinrichtung für die Fluide in eine Saugdruckregelkammer und

eine Saugdrucksteuerkammer mit Ein- und Auslaßöffnungen, die in Serie in dem zweiten Strömungsweg liegen, wobei die Einrichtung zur Ausbildung der Einwegströmung in der Saugdruckregelkammer in Serie mit deren Einlaßöffnung angeordnet ist und die Saugdruckregelkammer eine Flüssigkeit enthält, dadurch gekennzeichnet, daß diese Flüssigkeit eine sterile Flüssigkeit ist und daß das Auslaßende der Einrichtung zum Ausbilden der Einwegströmung unter dem Spiegel der sterilen Flüssigkeit und in Berührung mit dieser angeordnet ist.

2. Saugdruckregelkammer zur Verwendung beim Anlegen von Saugdruck an eine Körperhöhle über einen Absaugsammelbehälter, wobei die Saugdruckregelkammer folgende Teile umfaßt:

einen Einlaß und einen Auslaß, wobei der Einlaß für die Fluidströmung mit dem Sammelbehälter und der Auslaß für die Fluidströmung mit einer Saugdruckquelle verbindbar ist,

eine Einwegventileinheit mit Ein- und Auslaßenden, die beim Einlaß der Saugdruckregelkammer angeordnet ist, um eine Einwegströmung von Fluiden vom Einlaß in die Saugdruckregelkammer zu ermöglichen, und

eine in der Saugdruckregelkammer angeordnete Drucküberwachungseinrichtung zur Anzeige des Druckes in der Kammer, wobei die Kammer eine Flüssigkeit enthält, dadurch gekennzeichnet, daß diese Flüssigkeit eine sterile Flüssigkeit ist und daß das Auslaßende der Einwegventileinheit unter dem Spiegel der sterilen Flüssigkeit in Berührung mit dieser angeordnet ist.

3. Absaugdränagesystem nach Anspruch 1, bei dem die Einrichtung (46) zum Ausbilden einer Einwegströmung ein Elastomerglied mit einem Einlaß (46a) einem Auslaß (46b) ist, das zwei einander gegenüberliegende und normalerweise aneinanderliegende Flächen (55, 57) aufweist, die durch das Anlegen eines positiven Differentialdruckes zwischen Einlaß (46a) und Auslaß (46b) des Elastomergliedes voneinander trennbar sind.

4. Absaugdränagesystem nach Anspruch 1, bei dem die gasförmigen Fluide, welche die Einrichtung (46) zum Ausbilden einer Einwegströmung durchsetzen, durch die sterile Flüssigkeit (59) hindurch in den Umgebungsdruckraum entladen werden.

5. Gerät nach Anspruch 1 oder 4, bei dem die Saugdruckregelkammer (30) eine einstellbare Ventileinheit (38, 52, 54) aufweist, welche an die Auslaßöffnung (36) der Saugdruckregelkammer (30) angeschlossen ist, um an dieser Auslaßöffnung (36) eine einstellbare Drosselstelle (52, 54) vorzusehen, sowie eine Einrichtung (66) zum Entgasen der Saugdruckregelkammer (30) in den Umgebungsdruckraum enthält.

6. Absaugdränagesystem nach Anspruch 5, bei dem das Ventil (38) eine Gewindebohrung (52) sowie eine Schraube (54) aufweist, welche in die Gewindebohrung (52) eingreift.

7. Absaugdränagesystem nach Anspruch 1, bei dem die Saugdruckregelkammer (30) eine Strömungsdrosseleinrichtung (60, 62) aufweist, die mit der Auslaßöffnung (36) verbunden ist, sowie eine Einrichtung (66) zum Entgasen der Saugdruckregelkammer (30) in den Umgebungsdruckraum.

8. Absaugdränagesystem nach Anspruch 5 oder 7, bei dem die Drosselstelle (52, 54, 60, 62) so bemessen ist, daß die Gasströmung durch die Drosselstelle (52, 54, 60, 62) innerhalb eines absoluten Saugdruckbereiches von 0 bis 1/2 Atmosphären bei Schallgeschwindigkeit aufrecht erhalten wird.

9. Absaugdränagesystem nach Anspruch 7, bei dem die Einrichtung (66) zum Entgasen der Saugdruckregelkammer (30) etwas weiter als die Drosselstelle bemessen ist, so daß die Drosselstelle (60, 62) und die Einrichtung (66) zum Entgasen der Saugdruckregelkammer (30) im Zusammenwirken einen im wesentlichen konstanten negativen Druckpegel in der Saugdruckregelkammer (30) ergeben.

10. Absaugdränagesystem nach den Ansprüchen 1, 4, 5, 7 oder 9, das ferner eine Drucküberwachungseinrichtung (64, 68) enthält, die in der Saugdruckregelkammer (30) angeordnet ist, um eine Anzeige des Druckes in der Saugdruckregelkammer (30) zu bewirken.

11. Absaugdränagesystem nach Anspruch 9, bei dem die Drucküberwachungseinrichtung

ein sich verjüngendes hohles Rohr (64) mit einem ersten und einem zweiten Ende aufweist, wobei das erste Ende eine Öffnung (66) hat, die mit dem Umgebungsdruckraum außerhalb der Saugdruckregelkammer (30) in Verbindung steht, während das zweite Ende offen und innerhalb der Saugdruckregelkammer (30) angeordnet ist, und

einen beweglichen, druckempfindlichen Indikator (68) aufweist, der in diesem Rohr (64) angeordnet ist, um zwischen dem ersten und dem zweiten Ende des Rohres (64) eine Lage einzunehmen, die proportional der Differenz zwischen dem Umgebungsdruck und dem Druck innerhalb der Saugdruckregelkammer (30) ist.

12. Absaugdränagesystem nach Anspruch 11, bei dem der druckempfindliche Indikator eine Kugel (68) ist, die so ausgebilder und angeordnet ist, daß sie sich in dem Rohr (64) Abhängigkeit von der Druckdifferenz bewegt.

13. Absaugdränagesystem nach Anspruch 1, bei dem die Sammeleinrichtung (22) für die gedränten Fluide

einen starren äußeren Kanister (43),

eine Auskleidung (44) in Form eines Wegwerfbeutels innerhalb des Kanisters (43), wobei der Auskleidungsbeutel einen Deckel (24) hat,

une eine Einrichtung (40, 41, 49, 51, 70) zum Anlegen von negativem Druck seitens der Vakuumquelle sowohl an die Innenseite des Auskleidungsbeutels (44) als auch an die Innenseite des Kanisters (43) umfaßt, so daß der Auskleidungsbeutel (44) bei Anlegen von Vakkum an das System in gedehntem Zustand gehalten wird.

14. Absaugdränagesystem nach Anspruch 13, mit einem Einwegventil (53, 53a, 53b), das stromabwärts der Einrichtung (40, 49, 51, 70) zum Anlegen des negativen Druckes an die Innenseite des Kanisters (43) angeordnet ist, wodurch ein Vakuumverlust im Kanister (43) bei Beendigung des Saugdruckes seiten der Vakuumquelle vermieden wird.

15. Absaugdränagesystem nach Anspruch 1, bei dem die Saugdruckregelkammer (30) Mittel (34) zum lösbaren Befestigen derselben an der Sammeleinrichtung (22) für die gedränten Fluide aufweist.

16. Absaugdränagesystem nach Anspruch 11 mit einer Einrichtung (67, 69) zum Verhindern des Verschließens der Öffnung (66) mittels eines Fingers oder eines anderen Gegenstandes.

17. Saugdruckregelkammer nach Anspruch 2, bei welcher die Drucküberwachungseinrichtung (64, 66, 68)

ein sich verjüngendes hohles Rohr (64) mit einem ersten und einem zweiten Ende aufweist, wobei das erste Ende eine Öffnung (66) hat, die mit dem Umgebungsdruckraum außerhalb des Saugdruckregelkammer (30) in Verbindung steht, während das zweite Ende offen und innerhalb der Saugdruckregelkammer (30) angeordnet ist, und

einen beweglichen, druckempfindlichen Indikator (68) aufweist, der in diesem Rohr (64) angeordnet ist, um zwischen dem ersten und dem zweiten Ende des Rohres eine Lage einzunehmen, die proportional der Differenz zwischen dem Umgebungsdruck und dem Druck innerhalb der Saugdruckregelkammer (30) ist.

18. Saugdruckregelkammer nach Anspruch 2, die Mittel (34) zum lösbaren Befestigen der Saugdruckregelkammer (30) an dem Fluidsammelbehälter (22) aufweist.

19. Saugdruckregelkammer nach Anspruch 2 mit einer einstellbaren Ventileinheit (38, 52, 54), welche mit dem Auslaß (36) der Saugdruckregelkammer (30) verbunden ist, um an diesem Auslaß (36) eine einstellbare Drosselstellung zu bilden, und einer Einrichtung (68) zum Entgasen der Saugdruckregelkammer (30) in den Umgebungsdruckraum.

20. Saugdruckregelkammer nach Anspruch 2 mit einer Strömungsdrosseleinrichtung (60), die mit dem Auslaß (36) der Saugdruckregelkammer (30) verbunden ist, und mit einer Einrichtung (68) zum Entgasen der Saugdruckregelkammer (30) in den Umgebungsdruckraum.

21. Saugdruckregelkammer nach Anspruch 20, bei welcher die Strömungsdrosseleinrichtung (60) so bemessen ist, daß die Gasströmung über die Drossel (60) in einem absoluten Druckbereich von 0 bis 1/2 Atmosphären mit Schallgeschwindigkeit aufrechterhalten wird.

22. Saugdruckregelkammer nach Anspruch 2, bei welcher das Einwegventil (46) ein Elastomerglied aufweist, das zwei einander gegenüberliegende, normalerweise aneinander anliegende Flächen (55, 57) hat, die in Reaktion auf eine positive Druckdifferenz am Einwegventil (46) voneinander trennbar sind.

23. Saugdruckregelkammer nach Anspruch 19, bei dem das einstellbare Ventil eine Gewindebohrung (52) sowie eine Schraube (54) aufweist, welche in diese Gewindebohrung (52) eingreift.

24. Saugdruckregelkammer nach Anspruch 21, bei welcher die Einrichtung (66) zum Entgasen der Saugdruckregelkammer (30) etwas weiter bemessen ist als die Strömungsdrosseleinrichtung (60), so daß die Drosseleinrichtung (60) und die Einrichtung (66) zum Engasen der Saugdruckregelkammer (30) im Zusammenwirken einen im wesentlichen konstanten Pegel negativen Druckes innerhalb der Saugdruckregelkammer (30) ergeben.

**Revendications**

1. Système de drainage par aspiration destiné au drainage des fluides liquides et gazeux d'une cavité anatomique, le système comprenant

un dispositif collecteur des fluides drainés dans la cavité anatomique,

un dispositif destiné à établir un premier trajet de circulation de fluide entre la cavité anatomique et le dispositif collecteur des fluides drainés,

un dispositif destiné à établir un second trajet entre le dispositif collecteur des fluides et une source de vide,

un dispositif comprenant des extrémités d'entrée et de sortie et disposé dans le second trajet de circulation, de manière qu'il assure une circulation unidirectionnelle du fluide provenant du dispositif collecteur des fluides vers une chambre de réglage d'aspiration, et

une chambre de réglage d'aspiration ayant des ouvertures d'entrée et de sortie montées en série dans le second trajet de circulation, le dispositif destiné à former une circulation unidirectionnelle étant placé dans la chambre de réglage d'aspiration, en série avec l'ouverture d'entrée, la chambre de réglage d'aspiration contenant un liquide placé à l'intérieur, caractérisé en ce que le liquide est un liquide stérile, et en ce que l'extrémité de sortie du dispositif destiné à former un courant unidirectionnel est placée au-dessous du niveau du liquide stérile et au contact de celui-ci.

2. Chambre de réglage d'aspiration destinée à être utilisée pendant l'application d'une aspiration à une cavité anatomique par l'intermédiaire d'un récipient collecteur d'aspiration, la chambre de réglage d'aspiration comprenant

une entrée et une sortie, l'entrée étant destinée à être connectée de manière qu'elle communique avec le récipient collecteur, la sortie étant destinée à être connectée de manière qu'elle communique avec le récipient collecteur, la sortie étant destinée à être connectée afin qu'elle communique avec une source d'aspiration,

un clapet de retenue, ayant des extrémités d'entrée et de sortie et disposé à l'entrée de la chambre de réglage d'aspiration de manière qu'il forme un courant unidirectionnel de fluide allant de l'entrée à la chambre de réglage d'aspiration, et

un dispositif de contrôle de pression placé dans la chambre de réglage d'aspiration et destiné à donner une indication sur la pression qui y règne, la chambre de réglage d'aspiration contenant un liquide placé à l'intérieur, caractérisée en ce que le liquide est un liquide stérile et en ce que l'extrémité de sortie du clapet de retenue est placé au-dessous du niveau du liquide stérile, au contact de celui-ci.

3. Système de drainage par aspiration selon la revendication 1, dans lequel le dispositif (46) destiné à former un courant unidirectionnel est un organe élastomère ayant une entrée (46a) et une sortie (46b) et ayant deux surfaces opposées (55, 57) qui sont normalement en butée et qui peuvent être séparées à la suite de l'application d'une différence positive de pression de part et d'autre de l'organe élastomère, de son entrée (46a) à sa sortie (46b).

4. Système de drainage par aspiration selon la revendication 1, dans lequel les fluides gazeux qui circulent à partir du dispositif (46) destiné à former le courant unidirectionnel, sont évacués par l'intermédiaire du liquide stérile (59), jusqu'à la pression ambiante.

5. Appareil selon l'une des revendications 1 et 4, dans lequel la chambre (30) de réglage d'aspiration comporte une soupape réglable (38, 52, 54), reliée à l'ouverture de sortie (36) de la chambre (30) de réglage d'aspiration et destinée à former un rétrécissement réglable (52, 54) au niveau de l'ouverture de sortie (36), et un dispositif (66) destiné à relier la chambre (30) de réglage d'aspiration à la pression ambiante.

6. Système de drainage par aspiration selon la revendication 5, dans lequel le dispositif à soupape a un trou taraudé (52) et une vis (54) qui coopère avec le trou taraudé (52).

7. Système de drainage par aspiration selon la revendication 1, dans lequel la chambre (30) de réglage d'aspiration comporte un organe de réduction de débit (60, 62) relié à l'ouverture de sortie (36), et un dispositif (66) destiné à mettre la chambre (30) de réglage d'aspiration à la pression ambiante.

8. Système de drainage par aspiration selon l'une des revendications 5 et 7, dans lequel l'organe de rétrécissement (52, 54, 60, 62) a une dimension telle que le débit de gaz dans l'organe de rétrécissement (52, 54, 60, 62) reste à une vitesse sonique sur une plage de pressions absolues allant de zéro à la moitié d'une atmosphère.

9. Système de drainage par aspiration selon la revendication 7, dans lequel le dispositif (66) destiné à relier la chambre (30) de réglage d'aspiration à l'atmosphère a une dimension légèrement supérieure à celle de l'organe de réduction de débit, si bien que l'organe (60, 62) de réduction de débit et le dispositif (66) de mise de la chambre (30) de réglage d'aspiration à l'atmosphère assurent, en combinaison, l'obtention d'une dépression de niveau pratiquement constant à l'intérieur de la chambre (30) de réglage d'aspiration.

10. Système de drainage par aspiration selon l'une quelconque des revendications 1, 4, 5, 7 et 9, comprenant en outre un dispositif (64, 68) de contrôle de pression placé dans la chambre (30) de réglage d'aspiration et destiné à donner une indication sur la pression régnant dans la chambre (30) de réglage d'aspiration.

11. Système de drainage par aspiration selon la revendication 9, dans lequel le dispositif de contrôle de pression comporte

un tube creux et évasé (64) ayant une première et une seconde extrémité, la première extrémité ayant une ouverture (66) qui communique avec la pression ambiante régnant à l'extérieur de la chambre (30) de réglage d'aspiration, la seconde extrémité étant ouverte et étant placée dans la chambre (30) de réglage d'aspiration, et

un indicateur mobile (68) sensible à la pression, placé dans le tube (64) et destiné à prendre une position comprise entre la première et la seconde extrémité du tube (68), cette position dépendant de la différence entre la pression ambiante et la pression dans la chambre (30) de réglage d'aspiration.

12. Système de drainage par aspiration selon la revendication 11, dans lequel l'indicateur sensible à la pression est une bille (68) dont la construction et la disposition sont telles qu'elle se déplace dans le tube (64) d'après la différence de pression.

13. Système de drainage par aspiration selon la revendication 1, dans lequel le dispositif (22) collecteur des fluides drainés comporte

une enveloppe rigide externe (43),

un récepteur (44) formant un revêtement destiné à être jeté après usage, disposé à l'intérieur de l'enveloppe (43), ce récepteur ayant un couvercle (24), et

un dispositif (40, 41, 49, 51, 70 destiné à faire communiquer la dépression provenant de la source de vide à la fois à l'intérieur du récepteur (44) et à l'intérieur de l'enveloppe (43) afin que le récepteur (44) reste dilaté lorsque le vide est appliqué au système.

14. Système de drainage par aspiration selon la revendication 13, comprenant en outre un clapet de retenue (53, 53a, 53b) placé en aval du dispositif (40, 49, 51, 70) de mise en communication de la dépression avec l'intérieur de l'enveloppe (43), si bien que la perte du vide à l'intérieur de l'enveloppe (43) est évitée à la fin de l'aspiration par la source d'aspiration.

15. Système de drainage par aspiration selon la revendication 1, dans lequel la chambre (30) de réglage d'aspiration comporte un dispositif (34) de fixation temporaire de la chambre (30) de réglage d'aspiration sur le dispositif (22) collecteur des fluides drainés.

16. Système de drainage par aspiration selon la revendication 11, comprenant en outre un dispositif (67,69) destiné à empêcher le bouchage de ladite ouverture (66) par un doigt ou un autre objet analogue.

17. Chambre de réglage d'aspiration selon la revendication 2, dans laquelle le dispositif de contrôle de pression (64, 66, 68) comporte

un tube évasé (64) ayant une première et une seconde extrémité, la première extrémité ayant une ouverture (66) communiquant avec la pression ambiante à l'extérieur de la chambre (30) de réglage d'aspiration, la seconde extrémité débouchant dans la chambre (30) de réglage d'aspiration, et

un indicateur mobile (68), sensible à la pression, placé dans le tube (64) et destiné à prendre une position comprise entre la première et la seconde extrémité du tube, cette position dépendant de la différence entre la pression ambiante et la pression dans la chambre (30) de réglage d'aspiration.

18. Chambre de réglage d'aspiration selon la revendication 2, comprenant en outre un dispositif (34) de montage temporaire de la chambre (30) de réglage d'aspiration sur le récipient collecteur (22).

19. Chambre de réglage d'aspiration selon la revendication 2, comprenant en outre une soupape réglable (38, 52, 54) reliée à la sortie (36) de la chambre (30) de réglable au niveau de l'ouverture de sortie (36), et un dispositif (66) destiné à relier la chambre (30) de réglage d'aspiration à la pression ambiante.

20. Chambre de réglage d'aspiration selon la revendication 2, comprenant en outre un organe (60) de rétrécissement relié à la sortie (36) de la chambre (30) de réglage d'aspiration, et un dispositif (66) destiné à mettre la chambre (30) de réglage d'aspiration à la pression ambiante.

21. Chambre de réglage d'aspiration selon la revendication 20, dans laquelle l'organe de rétrécissement (60) a une dimension telle que le courant de gaz circulant dans l'organe de rétrécissement (60) garde une vitesse sonique dans toute une plage de pressions absolues allant de zéro à la moitie d'une atmosphère.

22. Chambre de réglage d'aspiration selon la revendication 2, dans laquelle le clapet de retenue (46) est un organe élastomère ayant deux surfaces opposées (55, 57) qui sont normalement en butée et qui sont séparable en présence d'une différence positive de pression établie de part et d'autre du clapet de retenue (46).

23. Chambre de réglage d'aspiration selon la revendication, 19, dans laquelle la soupape réglable comporte un trou taraudé (52) et une vis (54) coopérant avec ce trou taraudé (52).

24. Chambre de réglage d'aspiration selon la revendication 21, dans laquelle le dispositif (66) destiné à mettre la chambre (30) de réglage d'aspiration à l'atmosphère a une dimension légèrement supérieure à celle de l'organe de rétrécissement (60), si bien que l'organe de rétrécissement (60) et le dispositif (66) de mise de la chambre (30) de réglage d'aspiration à l'atmosphère donnent en combinaison une dépression de niveau pratiquement constant à l'intérieur de la chambre (30) de réglage d'aspiration.

FIG. 1

FIG. 2

TO PATIENT

TO VACUUM SOURCE

FIG. 2A

0 111 087

FIG. 3

FIG. 5

FIG. 4

2